# EUROPÄISCHE PATENTSCHRIFT

(11) **EP 3 583 653 B1**
(45) Veröffentlichungstag und Bekanntmachung des Hinweises auf die Patenterteilung: **16.12.2020**
(21) Anmeldenummer: 18711227.1
(22) Anmeldetag: 13.02.2018
(51) Int. Cl.: H01M 10/0567, H01G 11/64, H01G 11/60, H01G 11/62, H01M 10/0525, H01G 11/06

(54) **ELEKTROLYT-ZUSATZ FÜR LITHIUM-IONEN-BATTERIEN**
ELECTROLYTE-ADDITIVE FOR LITHIUM-ION BATTERY SYSTEMS
ADDITIF ÉLECTROLYTIQUE POUR BATTERIES LITHIUM-ION

(30) Priorität: 17.02.2017 DE 102017103334; 04.04.2017 DE 102017107257
(43) Veröffentlichungstag der Anmeldung: 25.12.2019
(73) Patentinhaber: Westfälische Wilhelms-Universität Münster, 48149 Münster (DE)
(72) Erfinder: WAGNER, Ralf, 25524 Itzehoe (DE); RÖSER, Stephan, 48161 Münster (DE); BROX, Sebastian, 48249 Dülmen (DE); LERCHEN, Andreas, 48149 Münster (DE); GLORIUS, Frank, 48149 Münster (DE); WINTER, Martin, 48149 Münster (DE)
(74) Vertreter: Michalski Hüttermann & Partner Patentanwälte mbB
(86) Internationale Anmeldenummer: PCT/EP2018/053555
(87) Internationale Veröffentlichungsnummer: WO 2018/149823

(56) Entgegenhaltungen:
- MIN SIK PARK ET AL: "Design of novel additives and nonaqueous solvents for lithium-ion batteries through screening of cyclic organic molecules: an ab initio study of redox potentials", PHYSICAL CHEMISTRY CHEMICAL PHYSICS., Bd. 16, Nr. 40, 1. Januar 2014 (2014-01-01), Seiten 22391-22398, XP055466509, GB ISSN: 1463-9076, DOI: 10.1039/C4CP03051F
- YUJIE LIANG ET AL: "Cationic Cobalt(III)-Catalyzed Aryl and Alkenyl C?H Amidation: A Mild Protocol for the Modification of Purine Derivatives", CHEMISTRY - A EUROPEAN JOURNAL, Bd. 21, Nr. 46, 29. September 2015 (2015-09-29), Seiten 16395-16399, XP055469460, DE ISSN: 0947-6539, DOI: 10.1002/chem.201503533
- JUHYEON PARK ET AL: "Comparative Catalytic Activity of Group?9 [Cp*M III ] Complexes: Cobalt-Catalyzed C?H Amidation of Arenes with Dioxazolones as Amidating Reagents", ANGEWANDTE CHEMIE INTERNATIONAL EDITION, Bd. 54, Nr. 47, 25. September 2015 (2015-09-25), Seiten 14103-14107, XP055469465, ISSN: 1433-7851, DOI: 10.1002/anie.201505820
- ZHANG ET AL: "A review on electrolyte additives for lithium-ion batteries", JOURNAL OF POWER SOURCES, ELSEVIER SA, CH, Bd. 162, Nr. 2, 22. November 2006 (2006-11-22), Seiten 1379-1394, XP027938606, ISSN: 0378-7753 [gefunden am 2006-11-22]

## Beschreibung

Die Erfindung betrifft das Gebiet der Lithium-Ionen-Batterien.

Lithium-Ionen-Batterien (Sekundärbatterien) stellen derzeit die führende Technologie auf dem Gebiet der wieder aufladbaren Batterien dar, insbesondere im Bereich der portablen Elektronik. Herkömmliche Lithium-Ionen-Batterien verwenden eine Anode aus Kohlenstoff, gewöhnlich Graphit. Der Ladungstransport erfolgt über einen Elektrolyten, der ein Lithiumsalz, welches in einem Lösungsmittel gelöst ist, umfasst. Im Stand der Technik sind verschiedene Elektrolyte und Leitsalze bekannt. Herkömmliche Lithium-Ionen-Batterien verwenden derzeit zumeist Lithiumhexafluorophosphat (LiPF₆).

Geeignete Elektrolyte zeichnen sich dadurch aus, dass die Ausbildung einer Festkörper-Elektrolyt-Phasengrenzfläche, der so genannten Solid Electrolyte Interphase (SEI) auf einer Elektrode induziert wird. Bei Graphit-Anoden kommt es dabei zu einer reduktiven Zersetzung des Elektrolyten und die Reaktionsprodukte können einen anhaftenden und elektronisch isolierenden, aber Lithiumionen-leitenden Film auf der Anode ausbilden. Die Solid Electrolyte Interphase verhindert im Folgenden, dass das Elektrodenmaterial weiter mit dem Elektrolyten reagiert und schützt hierdurch den Elektrolyten vor weiterer reduktiver Zersetzung und die Anode vor Zerstörung durch das Lösungsmittel. Insbesondere bei Verwendung von Graphit-Anoden ist die Ausbildung eines derartigen Films für einen sicheren Betrieb der Lithium-Ionen-Batterie notwendig.

Die reduktive Zersetzung des Lösemittels Propylencarbonat (IUPAC-Bezeichnung 4-Methyl-1,3-dioxolan-2-on) führt jedoch zu keiner Ausbildung einer Solid Electrolyte Interphase. So bewirkt die reduktiv induzierte Gasentwicklung innerhalb der Graphitschichten, induziert durch Co-Interkalation von Propylencarbonat, das Auseinanderbrechen ("Exfoliation") und die irreversible Zerstörung des Aktivmaterials. Dies limitiert die Nutzung von Propylencarbonat, trotz besserer thermischer und physikochemischer Eigenschaften gegenüber Ethylencarbonat, für die Lithium-Ionen-Technologie. Des Weiteren kann Propylencarbonat als Modellsystem für Elektrolyte dienen, die ebenfalls eine reduktive Zersetzung ohne SEI-Bildung und Exfoliation von Graphit zeigen.

Im Stand der Technik sind weitere Elektrolyte enthaltend Dioxolane als organische Lösungsmittel bekannt. Die Schrift DE 10 2010 020 992 A1 offenbart beispielsweise Lösungsmittel umfassend Ethylencarbonat, Propylencarbonat, Butylencarbonat und weitere wie 3-Methyl-1,3-oxazolidin-2-on, sowie Mischungen aus zwei oder mehrerer dieser Lösungsmittel. Weiter relevanter Stand der Technik ist in der Druckschrift MIN SIK PARK ET AL: "Design of novel additives and nonaqueous solvents for lithium-ion batteries through screening of cyclic organic molecules: an ab initio study of redox potentials", PHYSICAL CHEMISTRY CHEMICAL PHYSICS., Bd. 16, Nr. 40, 1. Januar 2014 (2014-01-01), Seiten 22391-22398, XP055466509, GB ISSN: 1463-9076 offenbart.

Es wurde bereits vorgeschlagen, die Exfoliation des Graphits und die reduktive Zersetzung des Lösungsmittels durch die Verwendung von hochkonzentrierten Elektrolyten zu unterbinden. Die Verwendung von hochkonzentrierten Elektrolyten, auch als "solvent-in-salt"-Elektrolyte bekannt, ist jedoch nicht wirtschaftlich, da dieser Ansatz ein Vielfaches der normalerweise benötigten Menge an Leitsalz erfordert. Des Weiteren erhöht sich durch die Konzentration (meist > 3 mol l⁻¹) die Viskosität des Elektrolyten stark, was zu einem merklichen Abfall der Leitfähigkeit und der Leistung der Batterie führt. Weiterhin ist zu erwarten, dass es bei einer Absenkung der Betriebstemperatur zur Unterschreitung des Löslichkeitsproduktes des Leitsalzes in der Elektrolytlösung kommt, welches zum Ausfallen des Salzes im Inneren der Batterien führt. Zudem erhöht sich bei gleichbleibendem Volumen und steigender Zugabe von Leitsalz die Dichte und daher die Gesamtmasse des Elektrolyten. Dies führt ebenfalls dazu, dass die spezifische Energiedichte (Ah kg⁻¹) der Batterie als Gesamtsystem abnimmt.

Weiter wurde die Verwendung von adäquaten Leistungsadditiven vorgeschlagen. Vor allem in kommerziellen Batterien wichtig sind hierbei Vinylencarbonat (VC) und Fluoroethylencarbonat (FEC). Das herkömmlich verwendete Additiv Vinylencarbonat kann jedoch nur bis zu einer Abschlussspannung von maximal 4,7 V in Lithium-Ionen-Batterien eingesetzt werden, da es sonst zu einer oxidativen Zersetzung kommt. Insbesondere für die Entwicklung von Hochvolt-Elektrolyten besteht daher ein Bedarf an SEI-bildenden Elektrolyt-Additiven mit erhöhter oxidativer Stabilität.

Der vorliegenden Erfindung lag daher die Aufgabe zu Grunde, einen Elektrolyten zur Verfügung zu stellen, der mindestens einen der vorgenannten Nachteile des Standes der Technik überwindet. Insbesondere lag der vorliegenden Erfindung die Aufgabe zu Grunde eine Verbindung bereit zu stellen, welche die Ausbildung einer Solid Electrolyte Interphase unterstützt.

Diese Aufgabe wird gelöst durch einen Elektrolyten für einen elektrochemischen Energiespeicher umfassend ein Elektrolytsalz und ein Lösemittel, wobei der Elektrolyt wenigstens eine Verbindung gemäß der allgemeinen Formel (1) wie nachstehend angegeben umfasst: 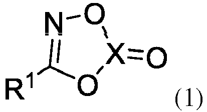 worin:
- X: ist C;
- R¹: ist ausgewählt aus der Gruppe umfassend CN, C₁-C₁₀-Alkyl, C₃-C₇-Cycloalkyl und/oder C₆-C₁₀-Aryl, wobei die Alkyl-, , Cycloalkyl- und Arylgruppen jeweils unsubstituiert oder einfach oder mehrfach mit wenigstens einem Substituenten ausgewählt aus der Gruppe umfassend F, C₁₋₄-Alkyl und/oder CN substituiert sind; und
wobei der Elektrolyt die Verbindung gemäß der allgemeinen Formel (1) in einem Bereich von ≥ 0,1 Gew.% bis ≤ 10 Gew.%, bezogen auf das Gesamtgewicht des Elektrolyten enthält.

Weitere vorteilhafte Ausgestaltungen der Erfindung ergeben sich aus den Unteransprüchen und den nebengeordneten Ansprüchen.

Überraschend wurde gefunden, dass Verbindungen gemäß der allgemeinen Formel (1) eine Solid Electrolyte Interphase (SEI) auf einer Graphit-Elektrode ausbilden. Durch Verwendung einer Verbindung gemäß der allgemeinen Formel (1) in Elektrolyten wird somit die Verwendung von Lösungsmitteln wie Propylencarbonat, die alleine keine Solid Electrolyte Interphase ausbilden, in elektrochemischen Energiespeichern wie Lithium-Ionen-Batterien ermöglicht. Es konnte festgestellt werden, dass die Verbindungen gemäß der allgemeinen Formel (1) dazu beitragen können, eine stabile Solid Electrolyte Interphase auszubilden, die über wenigstens 50 Lade- und Entladezyklen Graphitanoden vor einer Exfoliation sowie den Bulk-Elektrolyten vor der kontinuierlichen reduktiven Zersetzung schützen kann. Insbesondere ist dabei von Vorteil, dass die Zersetzung der Verbindungen gemäß der allgemeinen Formel (1) wie 3-Methyl-1,4,2-dioxoazol-5-on bei höheren Potentialen erfolgt als die von Vinylencarbonat. Die höhere oxidative Stabilität der SEI-bildenden Verbindungen der allgemeinen Formel (1) ermöglicht dadurch eine Verwendung in Hochvolt-Batterien.

In vorteilhafter Weise kann ein erfindungsgemäßer Elektrolyt somit als Hochvolt-Elektrolyt Verwendung finden. Ohne auf eine bestimmte Theorie festgelegt zu sein, wird angenommen, dass eine Polymerisierungsreaktion unter Einbeziehen der Doppelbindung der 1,4,2-Dioxoazol-5-on-Derivate gemäß der allgemeinen Formel (1) an dem vermuteten Reaktionsmechanismus der Bildung der SEI beteiligt ist, und damit wesentlich für die vorteilhaften Eigenschaften der Verbindungen ist.

Der Begriff "C₁-C₁₀-Alkyl" umfasst, wenn nicht anders angegeben, geradkettige oder verzweigte Alkylgruppen mit 1 bis 10 Kohlenstoffatomen. Unter dem Begriff "C₆-C₁₀-Aryl" sind aromatische Reste mit 6 bis 10 Kohlenstoff-Atomen zu verstehen. Der Begriff "Aryl" umfasst bevorzugt Carbocyclen. C₆-C₁₀-Arylgruppen sind vorzugsweise ausgewählt aus der Gruppe umfassend Phenyl und/oder Naphthyl, bevorzugt Phenyl. C₃-C₇-Cycloalkylgruppen sind vorzugsweise ausgewählt aus der Gruppe umfassend Cyclopentyl und/oder Cyclohexyl. C₁-C₅-Alkoxygruppen sind vorzugsweise ausgewählt aus der Gruppe umfassend Methoxy, Ethoxy, lineares oder verzweigtes Propoxy, Butoxy und/oder Pentoxy.

Der Substituent X ist Kohlenstoff.

Der Substituent R¹ ist vorzugsweise ausgewählt aus der Gruppe umfassend CN, C₁-C₅-Alkyl, C₁-C₅-Alkoxy, C₅-C₆-Cycloalkyl und/oder Phenyl, wobei die Alkyl-, Alkoxy-, Cycloalkyl-und Phenylgruppen jeweils unsubstituiert oder einfach oder mehrfach mit Fluor substituiert sind. Fluorierte Substituenten R¹ können die Ausbildung der Solid Electrolyte Interphase unterstützen. Insbesondere kann durch eine Fluorierung die Stabilität des Elektrolytadditivs gezielt an die Anforderungen einer Lithium-Ionen-Batterie angepasst werden. In bevorzugten Ausführungsformen ist R¹ ausgewählt aus der Gruppe umfassend CN und/oder unsubstituiertes oder einfach oder mehrfach mit Fluor substituiertes C₁-C₅-Alkyl oder Phenyl. Derartige Gruppen R¹ können in vorteilhafter Weise die Reaktionen der Doppelbindung, die zur Ausbildung einer Solid Electrolyte Interphase (SEI) auf einer Elektrode führen, unterstützen. C₁-C₅-Alkylgruppen sind insbesondere ausgewählt aus der Gruppe umfassend Methyl, Ethyl, Propyl, Isopropyl, Butyl, Isobutyl, tert-Butyl, Pentyl, Isopentyl, tert-Pentyl und/oder Neopentyl. In weiter bevorzugten Ausführungsformen ist R¹ ausgewählt aus der Gruppe umfassend CN, tert-Butyl, Phenyl und/oder unsubstituiertes oder einfach oder mehrfach mit Fluor substituiertes C₁-C₂-Alkyl. In besonders bevorzugten Ausführungsformen ist R¹ ausgewählt aus der Gruppe umfassend CH₃, CF₃, CN, tert-Butyl und/oder Phenyl.

Ist R¹ eine Gruppe -CO-O-R², ist R² vorzugsweise ausgewählt aus der Gruppe umfassend C₁-Cs-Alkyl, C₅-C₆-Cycloalkyl und/oder Phenyl.

Bevorzugt umfasst der Elektrolyt wenigstens eine Verbindung gemäß der allgemeinen Formel (1) worin:
- X: ist C;
- R¹: ist ausgewählt aus der Gruppe umfassend CN, C₁-C₅-Alkyl, C₅-C₆-Cycloalkyl, und/oder Phenyl, wobei die Alkyl-, Cycloalkyl- und Phenylgruppen jeweils unsubstituiert oder einfach oder mehrfach mit Fluor substituiert sind.

Besonders bevorzugt umfasst der Elektrolyt wenigstens eine Verbindung gemäß der allgemeinen Formel (1) worin X Kohlenstoff ist und/oder R¹ ausgewählt ist aus der Gruppe umfassend CN, Phenyl und/oder unsubstituiertes oder einfach oder mehrfach mit F substituiertes C₁-C₅-Alkyl, insbesondere C₁-C₂-Alkyl. In vorteilhafter Weise führen kleine Alkylgruppen zu Verbindungen, die zur effizienten Ausbildung einer Solid Electrolyte Interphase beitragen können. In bevorzugten Ausführungsformen ist X Kohlenstoff und R¹ ist ausgewählt aus der Gruppe umfassend CH₃, CF₃, CN, tert-Butyl und/oder Phenyl.

Besonders bevorzugt ist X Kohlenstoff und der Elektrolyt umfasst wenigstens ein 1,4,2-Dioxoazol-5-on-Derivat gemäß der allgemeinen Formel (2) 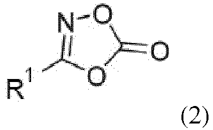 worin:
- R¹: ist ausgewählt aus der Gruppe umfassend CN, C₁-C₅-Alkyl, C₅-C₆-Cycloalkyl, und/oder Phenyl, wobei die Alkyl-, Cycloalkyl- und Phenylgruppen jeweils unsubstituiert oder einfach oder mehrfach mit Fluor substituiert sind.

Bevorzugt sind 1,4,2-Dioxoazol-5-on-Derivate worin R¹ ausgewählt ist aus der Gruppe umfassend CN und/oder unsubstituiertes oder einfach oder mehrfach mit Fluor substituiertes C₁-C₅-Alkyl oder Phenyl, insbesondere ausgewählt aus der Gruppe umfassend CN, tert-Butyl, Phenyl und/oder unsubstituiertes oder einfach oder mehrfach mit Fluor substituiertes C₁-C₂-Alkyl. In vorteilhafter Weise können insbesondere 1,4,2-Dioxoazol-5-on-Derivate in Elektrolyten eine stabile Solid Electrolyte Interphase ausbilden, die über eine Zyklisierdauer von wenigstens 50 Lade- und Entladezyklen Graphitanoden vor einer Exfoliation und den Bulk-Elektrolyten vor der kontinuierlichen Reduktion schützen kann. Ein besonders bevorzugtes 1,4,2-Dioxoazol-5-on-Derivat ist 3-Methyl-1,4,2-dioxoazol-5-on.

Es konnte gezeigt werden, dass ein Elektrolyt enthaltend 3-Methyl-1,4,2-dioxoazol-5-on ein weites oxidatives Stabilitätsfenster aufweist, und aufgrund dieser hohen Stabilität damit auch für Anwendungen mit Hochvoltelektrodenmaterialien verwendbar ist. Dies verdeutlicht einen besonderen Vorteil, da herkömmliche SEI-Bildner es nicht erlauben Graphit-Anoden mit Hochvoltkathodenmaterialien in Lithium-Ionen-Batterien mit Abschaltpotentialen höher als 4,5 V vs. Li/Li⁺ zu betreiben. Die erfindungsgemäßen Verbindungen sind als Additiv in Elektrolyten mit gängigen Elektrodenmaterialien wie Li(Ni_{1/3}Mn_{1/3}Co_{1/3})O₂ (NMC(111))-Kathoden, LiFePO₄ (LFP), LiMPO₄ (M = Mn, Ni, Co), LiMn₂O₄ (LMO), LiNiₓMn_{y}O₄ (LNMO), Li(NiₓCo_{y}Mn_{z})O₂ (x+y+z=1) (NMC(XYZ)), (Li₂MnO₃)ₓ(LiMO₂)₁₋ₓ und Li(NiₓCo_{y}Al_{z})O₂ (x+y+z=1) (NCA) verwendbar.

Die Verbindungen gemäß der allgemeinen Formel (1) sind kommerziell erhältlich oder nach dem Fachmann geläufigen Verfahren herstellbar.

Erfindungsgemäß enthält der Elektrolyt die Verbindung gemäß der allgemeinen Formel (1) in einem Bereich von ≥ 0,1 Gew.-% bis ≤ 10 Gew.-%, vorzugsweise im Bereich von ≥ 0,5 Gew.-% bis ≤ 7 Gew.-%, bevorzugt im Bereich von ≥ 3 Gew.-% bis ≤ 5 Gew.-%, bezogen auf das Gesamtgewicht des Elektrolyten. Angaben in Gew.-% sind jeweils bezogen auf ein Gesamtgewicht des Elektrolyten von 100 Gew.-%. Angaben in Prozent entsprechen, wenn nicht abweichend angegeben, Angaben in Gew.-%.

In vorteilhafter Weise kann ein Elektrolyt umfassend derartige Anteile einer Verbindung gemäß der allgemeinen Formel (1), insbesondere 1,4,2-Dioxoazol-5-on-Derivate wie 3-Methyl-1,4,2-dioxoazol-5-on, eine sehr gute Bildung einer Solid Electrolyte Interphase zeigen. In vorteilhafter Weise können Anteile im Bereich von ≥ 3 Gew.-% bis ≤ 5 Gew.-%, bezogen auf das Gesamtgewicht des Elektrolyten, bereits für eine effektive Passivierung von Graphit ausreichen. Solcherart geringe Gewichtsanteile eines Additivs erlauben eine wirtschaftliche kommerzielle Anwendung.

Weiter konnte festgestellt werden, dass bereits Elektrolyte enthaltend 2 Gew.-% 3-Methyl-1,4,2-dioxoazol-5-on, bezogen auf das Gesamtgewicht des Elektrolyten, eine sehr gute Zyklenstabilität bei tiefen Temperaturen aufwiesen. In vorteilhafter Weise sind die Verbindungen der Formel (1) wie 3-Methyl-1,4,2-dioxoazol-5-on für Tieftemperaturanwendungen gut geeignet und Lithium-Ionen-Batterien mit 3-Methyl-1,4,2-dioxoazol-5-on als Additiv können insbesondere bei tiefen Temperaturen mit einer guten Zyklenstabilität betrieben werden.

Der Elektrolyt weist neben wenigstens einem Elektrolytsalz, vorzugsweise einem Lithiumsalz, und wenigstens einer Verbindung gemäß der allgemeinen Formel (1) ein Lösemittel auf. Das Lösemittel dient vorzugsweise als Lösungsmittel für das Elektrolyt- bzw. Lithiumsalz.

In bevorzugten Ausführungsformen umfasst der Elektrolyt ein Lösemittel ausgewählt aus der Gruppe umfassend ein nicht-fluoriertes oder teilfluoriertes organisches Lösemittel, eine ionische Flüssigkeit, eine Polymermatrix und/oder Mischungen davon.

Vorzugsweise umfasst der Elektrolyt ein organisches Lösemittel, insbesondere ein cyclisches oder lineares Carbonat. In bevorzugten Ausführungsformen ist das organische Lösemittel ausgewählt aus der Gruppe umfassend Ethylencarbonat, Ethylmethylcarbonat, Propylencarbonat, Dimethylcarbonat, Diethylcarbonat, Acetonitril, Propionitril, 3-Methoxypropionitril, Glutaronitril, Adiponitril, Pimelonitril, gamma-Butyrolacton, gamma-Valerolacton, Dimethoxyethan, 1,3-Dioxolan, Methylacetat, Ethylacetat, Ethylmethansulfonat, Dimethylmethylphosphonat, lineares oder zyklisches Sulfon wie Ethylmethylsulfon oder Sulfolan, symmetrische oder nicht-symmetrische Alkylphosphate und/oder deren Mischungen. Weitere bevorzugte organische Lösemittel sind symmetrische oder nicht symmetrische Organosilane und/oder Siloxane. Bevorzugt sind ebenfalls Mischungen von symmetrischen oder nicht symmetrischen Organosilanen und/oder Siloxanen mit einem oder mehreren Lösemitteln ausgewählt aus der Gruppe umfassend Ethylencarbonat, Ethylmethylcarbonat, Propylencarbonat, Dimethylcarbonat, Diethylcarbonat, Acetonitril, Propionitril, 3-Methoxypropionitril, Glutaronitril, Adiponitril, Pimelonitril, gamma-Butyrolacton, gamma-Valerolacton, Dimethoxyethan, 1,3-Dioxolan, Methylacetat, Ethylacetat, Ethylmethansulfonat, Dimethylmethylphosphonat, lineares oder zyklisches Sulfon wie Ethylmethylsulfon oder Sulfolan und/oder symmetrische oder nicht-symmetrische Alkylphosphate.

Der Elektrolyt kann insbesondere Lösemittel wie Propylencarbonat umfassen, die ohne Additiv nicht zu einer Ausbildung einer Solid Electrolyte Interphase führen. Für die Verwendung mit diesen Lösungsmitteln ist ein Zusatz der erfindungsgemäßen Verbindungen zur Ausbildung einer effektiven Solid Electrolyte Interphase besonders vorteilhaft. In vorteilhafter Weise kann unter Verwendung einer Verbindung der allgemeinen Formel (1) ein Elektrolyt umfassend Propylencarbonat als Lösungsmittel eine vergleichbare oxidative Stabilität und eine ebenso effiziente SEI-Bildung aufweisen, wie bekannte Elektrolyte, die ohne Beimischung weiterer Verbindungen eine Solid Electrolyte Interphase ausbilden können. Der Elektrolyt umfasst vorzugsweise Propylencarbonat, oder eine Mischung umfassend Propylencarbonat und wenigstens ein weiteres organisches Lösemittel ausgewählt aus der Gruppe umfassend Ethylencarbonat, Ethylmethylcarbonat, Dimethylcarbonat, Diethylcarbonat, gamma-Butyrolacton, gamma-Valerolacton, Dimethoxyethan, 1,3-Dioxolan, Methylacetat, Ethylacetat, Ethylmethansulfonat, Dimethylmethylphosphonat, lineares oder cyclisches Sulfon wie Ethylmethylsulfon oder Sulfolan und/oder symmetrische oder nicht-symmetrische Alkylphosphate. Der Elektrolyt umfasst weiter vorzugsweise eine Mischung umfassend Propylencarbonat und wenigstens ein weiteres organisches Lösemittel ausgewählt aus symmetrischen oder nicht symmetrischen Organosilanen und Siloxanen.

Der Elektrolyt kann auch ein Polymerelektrolyt, beispielsweise ausgewählt aus der Gruppe umfassend Polyethylenoxid, Polyacrylnitril, Polyvinylchlorid, polyvinylidenfluorid, poly(vinylidenfluorid-co-hexafluoropropylen) und/oder Polymethylmethacrylat, oder ein Gel-Polymer-Elektrolyt umfassend ein Polymer und ein vorgenanntes organisches Lösungsmittel sein. Ebenfalls kann der Elektrolyt eine ionische Flüssigkeit sein.

Der erfindungsgemäße Elektrolyt weist neben einem Lösungsmittel und wenigstens einer Verbindung gemäß der allgemeinen Formel (1) wenigstens ein Elektrolytsalz, insbesondere ein Lithiumsalz auf.

In bevorzugten Ausführungsformen ist das Elektrolytsalz ausgewählt aus der Gruppe umfassend LiPF₆, LiBF₄, LiAsF₆, LiSbF₆, LiClO₄, LiPtCl₆, LiN(SO₂F)₂, LiN(SO₂CF₃)₂, LiN(SO₂C₂F₅)₂, LiC(SO₂CF₃)₃, LiB(C₂O₄)₂, LiBF₂(C₂O₄) und/oder LiSO₃CF₃. Bevorzugt ist das Lithiumsalz LiPF6. Die Konzentration des Lithiumsalzes im Elektrolyten kann in üblichen Bereichen liegen, beispielsweise im Bereich von ≥ 1,0 M bis ≤ 1,2 M.

In einer bevorzugten Ausführungsform umfasst der Elektrolyt eine Verbindung gemäß der allgemeinen Formel (1), insbesondere ein 1,4,2-Dioxoazol-5-on-Derivat wie 3-Methyl-1,4,2-dioxoazol-5-on, wenigstens ein Lithiumsalz, vorzugsweise LiPF₆, und Propylencarbonat oder eine Mischung organischer Lösemittel umfassend Propylencarbonat als Lösungsmittel. Der Elektrolyt ist beispielsweise herstellbar durch Einbringen des Lithiumsalzes in das Lösemittel und Zufügen der wenigstens einen Verbindung gemäß der allgemeinen Formel (1). Alternativ kann die Verbindung gemäß der allgemeinen Formel (1) zunächst mit dem Lösungsmittel gemischt werden und dann das Lithiumsalz hinzugegeben werden.

Der Elektrolyt kann weiterhin wenigstens ein Additiv enthalten, insbesondere ausgewählt aus der Gruppe umfassend SEI-Bildner und/oder Flammschutzmittel. Beispielsweise kann der Elektrolyt Verbindung gemäß der allgemeinen Formel (1) sowie einen weiteren SEI-Bildner enthalten. Geeignete Additive sind beispielsweise ausgewählt aus der Gruppe umfassend Fluorethylencarbonat, Chlorethylencarbonat, Vinylencarbonat, Vinylethylencarbonat, Ethylensulfit, Propansulton, Propensulton, Sulfite, vorzugsweise Dimethylsulfit und Propylensulfit, Ethylensulfat, Propylensulfat, Methylenmethandisulfonat, Trimethylensulfat, optional mit F, Cl oder Br substituierte Butyrolactone, Phenylethylencarbonat, Vinylacetat und/oder Trifluorpropylencarbonat. Beispielsweise kann der Elektrolyt Verbindung gemäß der allgemeinen Formel (1) sowie einen weiteren SEI-Bildner ausgewählt aus der Gruppe umfassend Vinylcarbonat, Fluorethylencarbonat und/oder Ethylensulfat enthalten. Diese Verbindungen können die Batterieleistung, beispielsweise die Kapazität, die Langzeitstabilität oder die Zyklen-Lebensdauer, verbessern.

Der Elektrolyt eignet sich insbesondere für eine Batterie oder einen Akkumulator, insbesondere als Elektrolyt für eine Lithium-Ionen-Batterie oder einen Lithium-Ionen-Akkumulator.

Ein weiterer Gegenstand der vorliegenden Erfindung betrifft einen elektrochemischen Energiespeicher, insbesondere einen Superkondensator oder Lithium-Ionen-basierten elektrochemischen Energiespeicher, umfassend einen erfindungsgemäßen, oben beschriebenen, Elektrolyten. Der Begriff "Energiespeicher" umfasst im Sinne der vorliegenden Erfindung primäre und sekundäre elektrochemische Energiespeichervorrichtungen, also Batterien (Primärspeicher) und Akkumulatoren (Sekundärspeicher). Im allgemeinen Sprachgebrauch werden Akkumulatoren häufig mit dem vielfach als Oberbegriff verwendeten Terminus "Batterie" bezeichnet. So wird der Begriff Lithium-Ionen-Batterie synonym zu Lithium-Ionen-Akkumulator verwendet, wenn nicht abweichend angegeben. Der Begriff "elektrochemische Energiespeicher" umfasst im Sinne der vorliegenden Erfindung auch elektrochemische Kondensatoren (englisch: electrochemical capacitors) oder Superkondensatoren (englisch: Supercapacitors, kurz Supercaps). Elektrochemische Kondensatoren, in der Literatur auch als Doppelschichtkondensatoren oder Superkondensatoren bezeichnet, sind elektrochemische Energiespeicher, die sich gegenüber Batterien durch eine deutlich höhere Leistungsdichte, gegenüber konventionellen Kondensatoren durch eine um Größenordnungen höhere Energiedichte auszeichnen. Lithium-Ionen-basierte elektrochemische Energiespeicher sind vorzugsweise ausgewählt aus der Gruppe umfassend Lithium-Ionen-Batterien, Lithium-Ionen-Akkumulatoren und Polymer-Batterien. Bevorzugt ist der Energiespeicher eine Lithium-Ionen-Batterie bzw. ein Lithium-Ionen Akkumulator. Weiter bevorzugt ist der elektrochemische Energiespeicher ein elektrochemischer Kondensator insbesondere Superkondensator. Es konnte gezeigt werden, dass die ausgebildete Festkörper-Elektrolyt-Phasengrenzfläche auf einer Graphit-Anode über wenigstens 50 Zyklen stabil war. Dies ermöglicht einen wirtschaftlichen Betrieb wiederaufladbarer Batterien.

Ein weiterer Gegenstand der Erfindung betrifft ein Verfahren zur Ausbildung einer Festkörper-Elektrolyt-Phasengrenzfläche auf einer Elektrode einer elektrochemischen Zelle umfassend eine Anode, eine Kathode und einen Elektrolyten, wobei man die Zelle unter Verwendung des erfindungsgemäßen, oben beschriebenen, Elektrolyten betreibt. Vorzugsweise wird die Festkörper-Elektrolyt-Phasengrenzfläche auf der Anode insbesondere einer Graphit-Anode, der negativen Elektrode, ausgebildet. Es hat sich bei der Betrachtung von Batterien eingebürgert, den Entladevorgang als Definition für die Begrifflichkeiten Anode und Kathode zu verwenden.

Ein weiterer Gegenstand der Erfindung betrifft die Verwendung einer Verbindung gemäß der allgemeinen Formel (1) wie nachstehend angegeben: 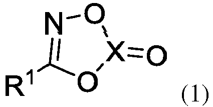 worin:
- X: ist C;
- R¹: ist ausgewählt aus der Gruppe umfassend CN, C₁-C₁₀-Alkyl, C₃-C₇-Cycloalkyl, und/oder C₆-C₁₀-Aryl, wobei die Alkyl-, , Cycloalkyl- und Arylgruppen jeweils unsubstituiert oder einfach oder mehrfach mit wenigstens einem Substituenten ausgewählt aus der Gruppe umfassend F, C₁₋₄-Alkyl und/oder CN substituiert sind,
in einem elektrochemischen Energiespeicher, insbesondere einem Superkondensator oder Lithium-Ionen-basierten elektrochemischen Energiespeicher, als Elektrolyt-Zusatz zur Ausbildung einer Festkörper-Elektrolyt-Phasengrenzfläche.

Die Verbindung gemäß der allgemeinen Formel (1) ist in erfindungsgemäßer Weise verwendbar als Elektrolyt-Zusatz, insbesondere als SEI-Bildner, insbesondere in Elektrolyten, die ohne Zusatz an Additiv keine SEI ausbilden.

Für die Beschreibung der Verbindung gemäß der allgemeinen Formel (1) wird auf die vorstehende Beschreibung verwiesen. Der Substituent X ist Kohlenstoff. R¹ ist vorzugsweise ausgewählt aus der Gruppe umfassend CN, C₁-C₅-Alkyl, C₅-C₆-Cycloalkyl und/oder Phenyl, wobei die Alkyl- Cycloalkyl- und Phenylgruppen jeweils unsubstituiert oder einfach oder mehrfach mit Fluor substituiert sind. In bevorzugten Ausführungsformen ist R¹ ausgewählt aus der Gruppe umfassend CN und/oder unsubstituiertes oder einfach oder mehrfach mit Fluor substituiertes C₁-C₅-Alkyl oder Phenyl. In weiter bevorzugten Ausführungsformen ist R¹ ausgewählt aus der Gruppe umfassend CN, tert-Butyl, Phenyl und/oder unsubstituiertes oder einfach oder mehrfach mit Fluor substituiertes C₁-C₂-Alkyl. In besonders bevorzugten Ausführungsformen ist R¹ ausgewählt aus der Gruppe umfassend CH₃, CF₃, CN, tert-Butyl und/oder Phenyl. Besonders bevorzugt ist X Kohlenstoff und/oder R¹ ist ausgewählt aus der Gruppe umfassend CN, Phenyl und/oder unsubstituiertes oder einfach oder mehrfach mit F substituiertes C₁-C₅-Alkyl insbesondere C₁-C₂-Alkyl. In bevorzugten Ausführungsformen ist X Kohlenstoff und R¹ ist ausgewählt aus der Gruppe umfassend CH₃, CF₃, CN, tert-Butyl und/oder Phenyl.

Besonders bevorzugt umfasst der Elektrolyt wenigstens ein 1,4,2-Dioxoazol-5-on-Derivat gemäß der allgemeinen Formel (2) worin R¹ ausgewählt ist aus der Gruppe umfassend CN, C₁-C₅-Alkyl, C₁-C₅-Alkoxy, C₅-C₆-Cycloalkyl, Phenyl und/oder -CO-O-R², wobei die Alkyl-, Alkoxy-, Cycloalkyl- und Phenylgruppen jeweils unsubstituiert oder einfach oder mehrfach mit Fluor substituiert sind, und/oder R² ausgewählt ist aus der Gruppe umfassend C₁-C₅-Alkyl, C₅-C₆-Cycloalkyl, und/oder Phenyl. Bevorzugt sind 1,4,2-Dioxoazol-5-on-Derivate, worin R¹ ausgewählt ist aus der Gruppe umfassend CN und/oder unsubstituiertes oder einfach oder mehrfach mit Fluor substituiertes C₁-C₅-Alkyl oder Phenyl, insbesondere ausgewählt aus der Gruppe umfassend CN, tert-Butyl, Phenyl und/oder unsubstituiertes oder einfach oder mehrfach mit Fluor substituiertes C₁-C₂-Alkyl. In vorteilhafter Weise können insbesondere 1,4,2-Dioxoazol-5-on-Derivate in Elektrolyten eine stabile Solid Electrolyte Interphase ausbilden, die über eine Zyklisierdauer von wenigstens 50 Lade- und Entladezyklen Graphitanoden vor einer Exfoliation sowie den Bulk-Elektrolyten vor der kontinuierlichen reduktiven Zersetzung schützen kann. Ein besonders bevorzugtes 1,4,2-Dioxoazol-5-on-Derivate ist 3-Methyl-1,4,2-dioxoazol-5-on.

Beispiele und Figuren, die der Veranschaulichung der vorliegenden Erfindung dienen, sind nachstehend angegeben.

Hierbei zeigen die Figuren:

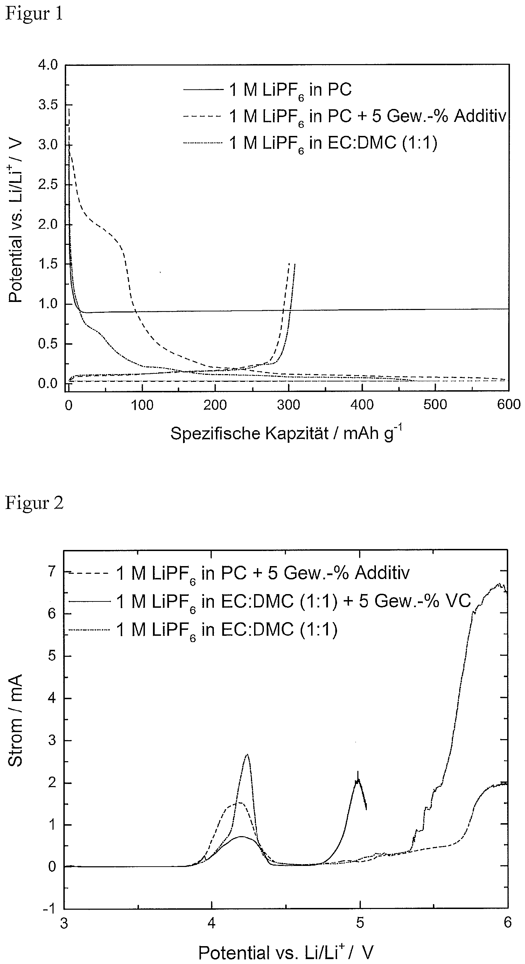

- Figur 1: zeigt den ersten Zyklus einer Graphit-Anode in einer Graphit/Lithium-Halbzelle unter Verwendung einer Elektrolytlösung gemäß einer Ausführungsform der Erfindung enthaltend 1 M LiPF6 und 5 Gew.-% 3-Methyl-1,4,2-dioxoazol-5-on (Additiv) in Propylencarbonat (PC), sowie von Vergleichselektrolyten enthaltend 1 M LiPF₆ in Propylencarbonat oder einer 1:1-Mischung von Ethylencarbonat und Dimethylcarbonat (EC:DMC). Aufgetragen ist das Potential gegen die spezifische Kapazität.
- Figur 2: zeigt das oxidative Stabilitätsfenster in LiMn₂O₄/Li Halbzellen des Elektrolyten enthaltend 1 M LiPF6 und 5 Gew.-% 3-Methyl-1,4,2-dioxoazol-5-on in Propylencarbonat (PC), sowie von Vergleichselektrolyten enthaltend 1 M LiPF6 in einer 1:1-Mischung von Ethylencarbonat und Dimethylcarbonat (EC:DMC) mit oder ohne 5 Gew.-% VC.

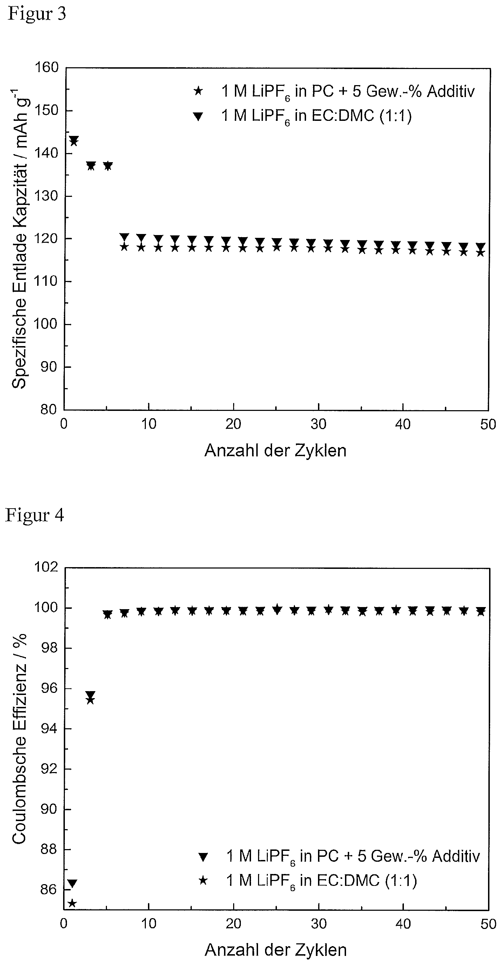

- Figur 3: zeigt die Konstantstromzyklisierung einer NMC/Graphit-Vollzelle unter Verwendung der Elektrolytlösung enthaltend 1 M LiPF6 und 5 Gew.-% 3-Methyl-1,4,2-dioxoazol-5-on (Additiv) in Propylencarbonat (PC), sowie eines Vergleichselektrolyten enthaltend 1 M LiPF6 in einer 1:1-Mischung von Ethylencarbonat und Dimethylcarbonat (EC:DMC) über 50 Zyklen. Aufgetragen ist die spezifische Entladekapazität in mAh g⁻¹ gegen die Anzahl der Zyklen. Nur jeder dritte Zyklus ist gezeigt.
- Figur 4: zeigt jeweils die Coulomb Effizienz aufgetragen gegen die Zyklenzahl für die NMC/Graphit-Vollzelle unter Verwendung der Elektrolytlösung enthaltend 1 M LiPF6 und 5 Gew.-% 3-Methyl-1,4,2-dioxoazol-5-on (Additiv) in Propylencarbonat (PC), sowie eines Vergleichselektrolyten enthaltend 1 M LiPF6 in einer 1:1-Mischung von Ethylencarbonat und Dimethylcarbonat (EC:DMC) über 50 Zyklen. Nur jeder dritte Zyklus ist gezeigt.

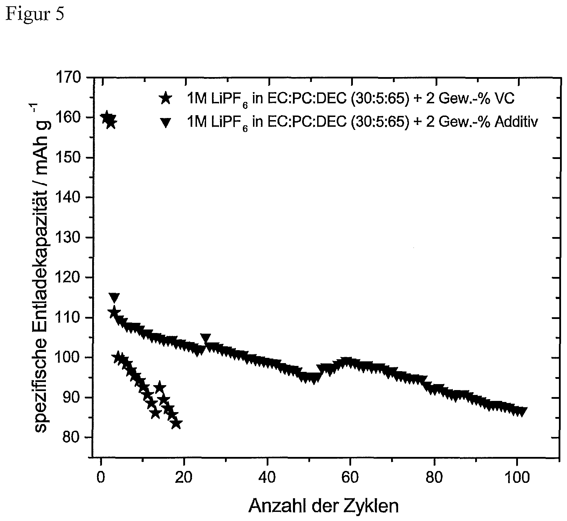

- Figur 5: zeigt die spezifische Entladekapazität gegen die Anzahl der Zyklen einer Konstantstromzyklisierung einer NMC622/Graphit-Vollzelle bei -20 °C (nach elektrochemischer Formierung bei 25 °C) unter Verwendung eines Elektrolyten enthaltend 1 M LiPF6 in einer 30:5:65 Gew.-%-Mischung von Ethylencarbonat, Propylencarbonat und Diethylcarbonat (EC:PC:DEC) und 2 Gew.-% 3-Methyl-1,4,2-dioxoazol-5-on (Additiv), sowie eines Vergleichselektrolyten enthaltend 2 Gew.-% Vinylencarbonat (VC).

### Beispiel 1

### Herstellung von 3-Methyl-1,4,2-dioxoazol-5-on

Die Synthese erfolgte wie von S. Chang et al. in J. Am. Chem. Soc. 2015, 137, Seiten 4534-4542 beschrieben. Hierzu wurden 50 mmol Acetohydroxamsäure (Sigma-Aldrich) in 500 mL Dichloromethan gelöst. Hierzu wurden 50 mmol 1,1'-Carbonyldiimidazol (Combi-Blocks) in einer Portion bei Raumtemperatur (20 °C ± 2 °C) hinzugegeben. Nach Rühren für 16 Stunden wurde die Reaktionsmischung mit 300 mL an 1 M HCl gequencht, drei Mal mit jeweils 150 mL Dichlormethan extrahiert und über Magnesiumsulfat getrocknet. Das Lösungsmittel wurde unter vermindertem Druck entfernt und 3-Methyl-1,4,2-dioxoazol-5-on wurde als farbloses, leicht-gelbliches Öl erhalten.

Das erhaltene Reaktionsprodukt wurde mittels ¹H- und ¹³C-NMR untersucht, wobei die ¹H-und ¹³C-Signale den erwarteten Werten für 3-Methyl-1,4,2-dioxoazol-5-on entsprachen.

### Beispiel 2

### Bestimmung der spezifischen Kapazität in einer Graphit/Li-Halbzelle

Für die Bestimmung der spezifischen Kapazität in Halbzellen wurde eine drei Elektroden Zelle (Swagelok®-Typ) verwendet. Als Elektroden wurden Graphit-Elektroden (SFG6L, Imerys SA) benutzt. Als Separator wurde ein Polymerfließ (Freudenberg SE, FS2226) verwendet. Lithiumfolie (Rockwood Lithium, Batteriereinheit) diente als Referenz- und Gegenelektrode.

Ein Elektrolyt enthaltend 1 M LiPF6 (BASF, Batterie Reinheit) und 5 Gew.-% 3-Methyl-1,4,2-dioxoazol-5-on erhalten aus Beispiel 1 wurde hergestellt, indem die benötigte Menge an LiPF6 in Propylencarbonat (PC, BASF, Batterie Reinheit) gelöst und die entsprechende Menge an 3-Methyl-1,4,2-dioxoazol-5-on zugegeben wurde. In gleicher Weise wurden Vergleichs-Elektrolyte enthaltend 1 M LiPF₆ in Propylencarbonat sowie einem Gemisch von Ethylencarbonat (BASF, Batterie Reinheit) und Dimethylcarbonat (BASF, Batterie Reinheit) (EC:DMC) in einem Gewichtsverhältnis von 1:1 hergestellt.

Untersucht wurden der erste Lade- und der erste Entladeprozess zwischen 0,025 V und 1,5 V vs. Li/Li⁺ bei einer C-Rate von 0,2C. Zusätzlich wurde das Potential für eine Stunde bei 0,025 V vs. Li/Li⁺ konstant gehalten. Die Figur 1 zeigt den ersten Zyklus der Graphit/Lithium-Halbzelle für die untersuchten Elektrolyte. Wie man der Figur 1 entnimmt, zeigte der Vergleichselektrolyten 1 M LiPF6 in Propylencarbonat keine reversible Kapazität. Das Zufügen von 5 Gew.-% an 3-Methyl-1,4,2-dioxoazol-5-on zu diesem Propylencarbonatbasierten Elektrolyten ermöglichte ein reversibles Zyklisieren, entsprechend der positiven Kontrolle des Vergleichselektrolyten enthaltend 1 M LiPF6 in einer 1:1-Mischung von Ethylencarbonat und Dimethylcarbonat.

### Beispiel 3

### Bestimmung der oxidativen elektrochemischen Stabilität in einer LMO/Li-Halbzelle

Die Bestimmung der oxidativen Stabilität der Elektrolyten in Halbzellen erfolgte mittels Linear Sweep-Voltammetrie. Bei diesem Verfahren erfolgt eine kontinuierliche Änderung der Elektrodenspannung (linear sweep). Hierfür wurde eine drei Elektroden Zelle (Swagelok®-Typ) unter Verwendung von Lithium-Mangan-oxid (LMO, Customcells GmbH) als Arbeitselektrode benutzt. Lithiumfolie (Rockwood Lithium, Batteriereinheit) diente als Referenz- und Gegenelektrode. Als Separator wurde ein Polymerfließ (Freudenberg SE, FS2226) verwendet. Zur Bestimmung der oxidativen Stabilität wurde das Potential zwischen Arbeits- und Referenzelektrode von der Leerlaufspannung auf 6 V erhöht. Die Vorschubgeschwindigkeit des Potentials betrug 0,05 mV s⁻¹.

Der Elektrolyt enthaltend 1 M LiPF6 und 5 Gew.-% 3-Methyl-1,4,2-dioxoazol-5-on erhalten aus Beispiel 1 wurde hergestellt, indem die benötigte Menge an LiPF6 in Propylencarbonat gelöst und die entsprechende Menge an 3-Methyl-1,4,2-dioxoazol-5-on zugegeben wurde, wie in Beispiel 2 beschrieben. In gleicher Weise wurden Vergleichs-Elektrolyte hergestellt, wobei ein Vergleichs-Elektrolyt 1 M LiPF6 in einem Gemisch von Ethylencarbonat und Dimethylcarbonat (EC:DMC) in einem Gewichtsverhältnis von 1:1 enthielt, und ein zweiter Vergleichs-Elektrolyt zusätzlich 5 Gew.-% des Additivs Vinylencarbonat (VC, UBE Industries).

Die Figur 2 zeigt das oxidative Stabilitätsfenster der Elektrolyte für ein Potential vs. Li/Li⁺ im Bereich von 3 V bis 6 V. Wie man der Figur 2 entnimmt, war der Elektrolyt enthaltend 5 Gew.-% an 3-Methyl-1,4,2-dioxoazol-5-on in Propylencarbonat bis 5,6 V vs. Li/Li⁺ stabil, die Elektrolyte in Ethylencarbonat/Dimethylcarbonat mit oder ohne Zusatz von 5 Gew.-% Vinylencarbonat jeweils bis 4,7 V und 5,3 V vs. Li/Li⁺.

Der erfindungsgemäße Elektrolyt enthaltend 5 Gew.-% an 3-Methyl-1,4,2-dioxoazol-5-on zeigt somit ein deutlich höheres oxidatives Stabilitätslimit als der Vinylencarbonat-basierende Elektrolyt. Dies weist darauf hin, dass 3-Methyl-1,4,2-dioxoazol-5-on später oxidiert wird als Vinylencarbonat, und somit eine bessere Hochvoltstabilität aufweist. Der erfindungsgemäße Elektrolyt enthaltend 5 Gew.-% an 3-Methyl-1,4,2-dioxoazol-5-on in Propylencarbonat zeigt auch eine deutlich höhere oxidative Stabilität als der Referenzelektrolyt 1 M LiPF6 in einem Gemisch von Ethylencarbonat und Dimethylcarbonat (EC:DMC). Entsprechend ist 3-Methyl-1,4,2-dioxoazol-5-on aufgrund seiner hohen oxidativen Stabilität insbesondere für Anwendungen mit Hochvoltelektrodenmaterialien verwendbar.

Somit kann eine effektive Passivierung von Graphit durch die Verwendung der erfindungsgemäßen Dioxazolon-Derivate in geringen Gewichtsanteilen im Elektrolyten erzielt werden. Dadurch, dass bereits 5 Gew.-% 3-Methyl-1,4,2-dioxoazol-5-on in Propylencarbonat für die Ausbildung einer Solid Electrolyte Interphase (SEI) auf Graphit-Elektroden ausreichen, ist die Verwendung von Dioxazolonen als Additiv für Propylencarbonat-basierte Elektrolyte auch wirtschaftlich.

### Beispiel 4

### Untersuchung der Langzeitzyklenstabilität unter Anlegen eines konstanten Stroms

Die Untersuchung der Langzeitzyklenstabilität erfolgte in Vollzellen eines Knopfzellaufbaus (Hohsen Corp., CR2032) unter Verwendung von Litihum-Nickel_{1/3}-Mangan_{1/3}-Cobalt_{1/3}-Oxid (NMC(111), Customcells GmbH) und Graphit Elektroden (Customcells GmbH). Als Separator wurde ein Polymerfließ (Freudenberg SE, FS2226) verwendet. Die Zyklisierungen wurden in einem Spannungsfenster von 4,2 V bis 2,8 V durchgeführt. Es wurden 2 Formierungszyklen gefahren bei 0,1C (inklusive Halten der Spannung bei 4,2V bis der Strom <0,05C ist), gefolgt von 3 Konditionierungszyklen bei 0,33C (inklusive Halten der Spannung bei 4,2V bis der Strom <0,05C ist und einem Warteschritt von einer Stunde), gefolgt von 95 1,0C Lade-/Entladezyklen. Die Messungen bei konstantem Strom wurden an einem Batterietester Series 4000 (Maccor) bei 20 °C ± 0,1°C durchgeführt.

Der Elektrolyt enthaltend 1 M LiPF6 und 5 Gew.-% 3-Methyl-1,4,2-dioxoazol-5-on erhalten aus Beispiel 1 wurde hergestellt, indem die benötigte Menge an LiPF6 in Propylencarbonat gelöst und die entsprechende Menge an 3-Methyl-1,4,2-dioxoazol-5-on zugegeben wurde, wie in Beispiel 2 beschrieben. Als Vergleichs-Elektrolyt wurde eine Lösung von 1 M LiPF₆ in einem Gemisch von Ethylencarbonat und Dimethylcarbonat (EC:DMC) in einem Gewichtsverhältnis von 1:1 hergestellt.

In Figur 3 ist die spezifische Entladekapazität der NMC/Graphit-Vollzelle gegen die Zyklenzahl und in Figur 4 die Coulomb Effizienz gegen die Zyklenzahl unter Verwendung der jeweiligen Elektrolyte gezeigt. Wie die Figur 3 zeigt, zeigte der Elektrolyt enthaltend 5 Gew.-% 3-Methyl-1,4,2-dioxoazol-5-on nur einen geringen Kapazitätsverlust über 50 Zyklen. Die Kapazitätserhaltung im Zyklus 50 bezogen auf den ersten Zyklus bei 1,0C (Zyklus 6) betrug 98,7%. Der Vergleichs-Elektrolyt 1 M LiPF6 in einem Gemisch von Ethylencarbonat und Dimethylcarbonat (EC:DMC) zeigte eine Kapazitätserhaltung von 98,1%.

Aus Figur 4 kann man weiter entnehmen, dass die Coulombsche Effizienz im ersten Zyklus 85,3% für den Elektrolyten enthaltend 5 Gew.-% 3-Methyl-1,4,2-dioxoazol-5-on betrug. Der Vergleichs-Elektrolyt 1 M LiPF6 in einem Gemisch von Ethylencarbonat und Dimethylcarbonat (EC:DMC) zeigte eine Coulombsche Effizienz im ersten Zyklus von 86,3%. Die Vollzelle mit dem Elektrolyten enthaltend 5 Gew.-% 3-Methyl-1,4,2-dioxoazol-5-on zeigte somit eine gute Zyklenstabilität, entsprechend dem Vergleichs-Elektrolyt 1 M LiPF6 in einem Gemisch von Ethylencarbonat und Dimethylcarbonat (EC:DMC).

Insgesamt zeigen die Ergebnisse, dass 3-Methyl-1,4,2-dioxoazol-5-on eine passivierende, Lithium-Ionen leitende Schutzschicht auf der Oberfläche von Graphit ausbildet, für Hochvoltanwendungen geeignet ist und Lithium-Ionen-Batterien mit einer guten Zyklenstabilität betrieben werden können.

### Beispiel 5

### Untersuchung des Zyklisierverhaltens bei tiefen Temperaturen

Die Untersuchung des Zyklisierverhaltens bei tiefen Temperaturen erfolgte in Vollzellen mit Knopfzellaufbau (Hohsen Corp., CR2032) mit Litihum-Nickel-Mangan-Cobalt-Oxid (NMC622, Customcells GmbH) und Graphit Elektroden (Customcells GmbH) unter Verwendung eines S240/P20 Separion-Separators. Die Zyklisierungen wurden in einem Spannungsfenster von 2,8-4,2 V durchgeführt. Die elektrochemische Formierung bei 2x 0,1C und der anschließende Ladeprozess bei 0,1C erfolgte bei einer Temperatur von 25 °C, die folgenden Lade-/Entladezyklen bei 0,3333C wurden bei einer Temperatur von -20 °C gefahren. Die Messungen wurden an einem Batterietester Series 4000 (Maccor) durchgeführt.

Als Elektrolyt wurden 90 µl einer Lösung enthaltend 1 M LiPF6 in einem Gemisch von Ethylencarbonat, Propylencarbonat und Diethylcarbonat (EC:PC:DEC) in einem Gewichtsverhältnis von 30:5:65 sowie 2 Gew.-% 3-Methyl-1,4,2-dioxoazol-5-on erhalten aus Beispiel 1 verwendet. Als Vergleichs-Elektrolyt wurde eine Lösung von 1 M LiPF6 in EC:PC:DEC enthaltend 2 Gew.-% des Additivs Vinylencarbonat (VC, UBE Industries) verwendet. Die Elektrolyte wurden hergestellt, indem die benötigte Menge an LiPF6 im Lösungsmittelgemisch gelöst und die entsprechende Menge an Additiv zugegeben wurde, wie in Beispiel 2 beschrieben.

Die Figur 5 zeigt die spezifische Entladekapazität bei -20 °C, nach vorheriger Formierung bei 25°C für 2 Zyklen, aufgetragen gegen die Anzahl der Zyklen für den Elektrolyten enthaltend 2 Gew.-% 3-Methyl-1,4,2-dioxoazol-5-on (Additiv), sowie des Vergleichselektrolyten enthaltend 2 Gew.-% Vinylencarbonat (VC).

Wie die Figur 5 zeigt, zeigte der Elektrolyt enthaltend 2 Gew.-% 3-Methyl-1,4,2-dioxoazol-5-on bei tiefen Temperaturen einen guten Kapazitätserhalt, wobei diese nach 100 Zyklen noch bei mehr als 80% der Kapazität von Zyklus 6 lag. Demgegenüber zeigt der Vergleichselektrolyt nur eine sehr geringe zyklische Lebensdauer von nur 20 Zyklen bis das EOL (End of Life) Kriterium von 80% der Kapazität von Zyklus 6 erreicht ist.

Dies zeigt, dass 3-Methyl-1,4,2-dioxoazol-5-on im Gegensatz zu Vinylencarbonat für Tieftemperaturanwendungen gut geeignet ist und Lithium-Ionen-Batterien mit 3-Methyl-1,4,2-dioxoazol-5-on als Additiv auch bei tiefen Temperaturen mit einer guten Zyklenstabilität betrieben werden können.

## Patentansprüche

1. Elektrolyt für einen elektrochemischen Energiespeicher umfassend ein Elektrolytsalz und ein Lösemittel, **dadurch gekennzeichnet, dass** der Elektrolyt wenigstens eine Verbindung gemäß der allgemeinen Formel (1) wie nachstehend angegeben umfasst: 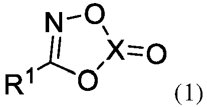 worin:
X ist C;
R¹ ist ausgewählt aus der Gruppe umfassend CN, C₁-C₁₀-Alkyl, C₃-C₇-Cycloalkyl und/oder C₆-C₁₀-Aryl, wobei die Alkyl-, Cycloalkyl- und Arylgruppen jeweils unsubstituiert oder einfach oder mehrfach mit wenigstens einem Substituenten ausgewählt aus der Gruppe umfassend F, C₁₋₄-Alkyl und/oder CN substituiert sind, und
wobei der Elektrolyt die Verbindung gemäß der allgemeinen Formel (1) in einem Bereich von ≥ 0,1 Gew.-% bis ≤ 10 Gew.-%, bezogen auf das Gesamtgewicht des Elektrolyten, enthält.

2. Elektrolyt nach Anspruch 1, **dadurch gekennzeichnet, dass** R¹ ausgewählt ist aus der Gruppe umfassend CN und/oder unsubstituiertes oder einfach oder mehrfach mit Fluor substituiertes C₁-C₅-Alkyl oder Phenyl.

3. Elektrolyt nach Anspruch 1 oder 2, **dadurch gekennzeichnet, dass** X Kohlenstoff ist und R¹ ausgewählt ist aus der Gruppe umfassend CH₃, CF₃, CN, tert-Butyl und/oder Phenyl.

4. Elektrolyt nach einem der vorhergehenden Ansprüche, **dadurch gekennzeichnet, dass** der Elektrolyt die Verbindung gemäß der allgemeinen Formel (1) im Bereich von ≥ 0,5 Gew.-% bis ≤ 7 Gew.-%, bevorzugt im Bereich von ≥ 3 Gew.-% bis ≤ 5 Gew.-%, bezogen auf das Gesamtgewicht des Elektrolyten, enthält.

5. Elektrolyt nach einem der vorhergehenden Ansprüche, **dadurch gekennzeichnet, dass** das Lösemittel ausgewählt ist aus der Gruppe umfassend ein nicht-fluoriertes oder teilfluoriertes organisches Lösemittel, eine ionische Flüssigkeit, eine Polymermatrix und/oder Mischungen davon.

6. Elektrolyt nach einem der vorhergehenden Ansprüche, **dadurch gekennzeichnet, dass** das Lösemittel ein organisches Lösemittel ist ausgewählt aus der Gruppe umfassend Ethylencarbonat, Ethylmethylcarbonat, Propylencarbonat, Dimethylcarbonat, Diethylcarbonat, Acetonitril, Propionitril, 3-Methoxypropionitril, Glutaronitril, Adiponitril, Pimelonitril, gamma-Butyrolacton, gamma-Valerolacton, Dimethoxyethan, 1,3-Dioxolan, Methylacetat, Ethylacetat, Ethylmethansulfonat, Dimethylmethylphosphonat, lineares oder zyklisches Sulfon, symmetrische oder nicht-symmetrische Alkylphosphate und/oder deren Mischungen.

7. Elektrolyt nach einem der vorhergehenden Ansprüche, **dadurch gekennzeichnet, dass** das Elektrolytsalz ausgewählt ist aus der Gruppe umfassend LiPF₆, LiBF4, LiAsF₆, LiSbF₆, LiClO₄, LiPtCl₆, LiN(SO₂F)₂, LiN(SO₂CF₃)₂, LiN(SO₂C₂F₅)₂, LiC(SO₂CF₃)₃, LiB(C₂O₄)₂, LiBF₂(C₂O₄) und/oder LiSO₃CF₃, bevorzugt LiPF6.

8. Elektrochemischer Energiespeicher, insbesondere Superkondensator oder Lithiumbasierter elektrochemischer Energiespeicher, umfassend einen Elektrolyten nach einem der Ansprüche 1 bis 7.

9. Verfahren zur Ausbildung einer Festkörper-Elektrolyt-Phasengrenzfläche auf einer Elektrode einer elektrochemischen Zelle umfassend eine Anode, eine Kathode und einen Elektrolyten, wobei man die Zelle unter Verwendung eines Elektrolyten nach einem der Ansprüche 1 bis 7 betreibt.

10. Verwendung einer Verbindung gemäß der allgemeinen Formel (1) wie nachstehend angegeben: 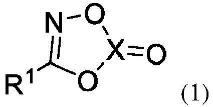 worin:
X ist C;
R¹ ist ausgewählt aus der Gruppe umfassend CN, C₁-C₁₀-Alkyl, C₃-C₇-Cycloalkyl und/oder C₆-C₁₀-Aryl, wobei die Alkyl-, Cycloalkyl- und Arylgruppen jeweils unsubstituiert oder einfach oder mehrfach mit wenigstens einem Substituenten ausgewählt aus der Gruppe umfassend F, C₁₋₄-Alkyl und/oder CN substituiert sind,
in einem elektrochemischen Energiespeicher, insbesondere einem Superkondensator oder Lithium-Ionen-basierten elektrochemischen Energiespeicher, als Elektrolyt-Zusatz zur Ausbildung einer Festkörper-Elektrolyt-Phasengrenzfläche.

## Claims

1. An electrolyte for an electrochemical energy storing device comprising an electrolyte salt and a solvent, **characterized in that** the electrolyte comprises at least one compound of the general formula (1) as indicated below: 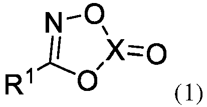 where:
X is C;
R¹ is selected from the group comprising CN, C₁-C₁₀-alkyl, C₃-C₇-cycloalkyl and/or C₆-C₁₀-aryl, where the alkyl, cycloalkyl and aryl groups are each unsubstituted or monosubstituted or polysubstituted by at least one substituent selected from the group comprising F, C₁₋₄-alkyl and/or CN; and
wherein the electrolyte contains the compound of the general formula (1) in an amount in the range from ≥ 0.1% by weight to ≤ 10% by weight, based on the total weight of the electrolyte.

2. The electrolyte according to claim 1, **characterized in that** R¹ is selected from the group comprising CN and/or C₁-C₅-alkyl or phenyl which are unsubstituted or monosubstituted or polysubstituted by fluorine.

3. The electrolyte according to claim 1 or 2, **characterized in that** X is carbon and R¹ is selected from the group comprising CH₃, CF₃, CN, tert-butyl and/or phenyl.

4. The electrolyte according to any one of the preceding claims, **characterized in that** the electrolyte contains the compound of the general formula (1) in an amount in the range from ≥ 0.5% by weight to ≤ 7% by weight, preferably in the range from ≥ 3% by weight to ≤ 5% by weight, based on the total weight of the electrolyte.

5. The electrolyte according to any one of the preceding claims, **characterized in that** the solvent is selected from the group comprising an unfluorinated or partially fluorinated organic solvent, an ionic liquid, a polymer matrix and/or mixtures thereof.

6. The electrolyte according to any one of the preceding claims, **characterized in that** the solvent is an organic solvent selected from the group comprising ethylene carbonate, ethyl methyl carbonate, propylene carbonate, dimethyl carbonate, diethyl carbonate, acetonitrile, propionitrile, 3-methoxypropionitrile, glutaronitrile, adiponitrile, pimelonitrile, gamma-butyrolactone, gamma-valerolactone, dimethoxyethane, 1,3-dioxolane, methyl acetate, ethyl acetate, ethyl methanesulfonate, dimethyl methylphosphonate, linear or cyclic sulfone, symmetrical or unsymmetrical alkyl phosphates and/or mixtures thereof.

7. The electrolyte according to any of the preceding claims, **characterized in that** the electrolyte salt is selected from the group comprising LiPF₆, LiBF₄, LiAsF₆, LiSbF₆, LiClO₄, LiPtCl₆, LiN(SO₂F)₂, LiN(SO₂CF₃)₂, LiN(SO₂C₂F₅)₂, LiC(SO₂CF₃)₃, LiB(C₂O₄)₂, LiBF₂(C₂O₄) and/or LiSO₃CF₃, preferably LiPF₆.

8. An electrochemical energy storing device, in particular supercapacitor or electrochemical energy storing device based on lithium, comprising an electrolyte as claimed in any one of claims 1 to 7.

9. A method for forming a solid electrolyte interphase on an electrode of an electrochemical cell comprising an anode, a cathode and an electrolyte, wherein the cell is operated using an electrolyte as claimed in any one of claims 1 to 7.

10. Use of a compound of the general formula (1) as indicated below: 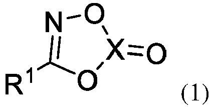 where:
X is C;
R¹ is selected from the group comprising CN, C₁-C₁₀-alkyl, C₃-C₇-cycloalkyl and/or C₆-C₁₀-aryl, where the alkyl, cycloalkyl and aryl groups are each unsubstituted or monosubstituted or polysubstituted by at least one substituent selected from the group comprising F, C₁₋₄-alkyl and/or CN,
in an electrochemical energy storing device, in particular a supercapacitor or an electrochemical energy storing device based on lithium ions, as an electrolyte additive for forming a solid electrolyte interphase.

## Revendications

1. Électrolyte pour un accumulateur d'énergie électrochimique comprenant un sel d'électrolyte et un solvant, **caractérisé en ce que** l'électrolyte comprend un composé selon la formule générale (1) telle qu'indiquée ci-après : 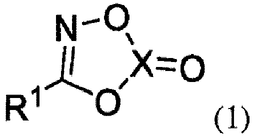 dans laquelle :
X est C ;
R¹ est choisi dans le groupe comprenant CN, alkyle en C₁-C₁₀, cycloalkyle en C₃-C₇ et/ou aryle en C₆-C₁₀, les groupes alkyle, cycloalkyle et aryle étant chacun non substitués ou substitués une fois ou plusieurs fois avec au moins un substituant choisi dans le groupe comprenant F, alkyle en C₁₋₄ et/ou CN, et
l'électrolyte contenant le composé selon la formule générale (1) dans une plage allant de ≥ 0, 1 % en poids à ≤ 10 % en poids, par rapport au poids total de l'électrolyte.

2. Électrolyte selon la revendication 1, **caractérisé en ce que** R¹ est choisi dans le groupe comprenant CN et/ou alkyle en C₁-C₅ ou phényle non substitué ou substitué une fois ou plusieurs fois avec fluor.

3. Électrolyte selon la revendication 1 ou 2, **caractérisé en ce que** X est un carbone et R¹ est choisi dans le groupe comprenant CH₃, CF₃, CN, tert-butyle et/ou phényle.

4. Électrolyte selon l'une quelconque des revendications précédentes, **caractérisé en ce que** l'électrolyte contient le composé selon la formule générale (1) dans la plage allant de ≥ 0,5 % en poids à ≤ 7 % en poids, de préférence dans la plage allant de ≥ 3 % en poids à ≤ 5 % en poids, par rapport au poids total de l'électrolyte.

5. Électrolyte selon l'une quelconque des revendications précédentes, **caractérisé en ce que** le solvant est choisi dans le groupe comprenant un solvant organique non fluoré ou partiellement fluoré, un liquide ionique, une matrice de polymère et/ou des mélanges de ceux-ci.

6. Électrolyte selon l'une quelconque des revendications précédentes, **caractérisé en ce que** le solvant est un solvant organique choisi dans le groupe comprenant le carbonate d'éthylène, le carbonate d'éthylméthyle, le carbonate de propylène, le carbonate de diméthyle, le carbonate de diéthyle, l'acétonitrile, le propionitrile, le 3-méthoxypropionitrile, le glutaronitrile, l'adiponitrile, le pimélonitrile, la gamma-butyrolactone, la gamme-valérolactone, le diméthoxyéthane, le 1,3-dioxolane, l'acétate de méthyle, l'acétate d'éthyle, le méthane-sulfonate d'éthyle, le phosphonate de diméthylméthyle, une sulfone linéaire ou cyclique, des phosphates d'alkyle symétriques ou non symétriques et/ou leurs mélanges.

7. Électrolyte selon l'une quelconque des revendications précédentes, **caractérisé en ce que** le sel d'électrolyte est choisi dans le groupe comprenant LiPF₆, LiBF₄, LiAsF₆, LiSbF₆, LiClO₄, LiPtCl₆, LiN (SO₂F)₂, LiN (SO₂CF₃)₂, LiN (SO₂C₂F₅)₂, LiC (SO₂CF₃)₃, LiB (C₂O₄)₂, LiBF₂ (C₂O₄) et/ou LiSO₃CF₃, de préférence LiPF₆.

8. Accumulateur d'énergie électrochimique, notamment supercondensateur ou accumulateur d'énergie électrochimique à base de lithium, comprenant un électrolyte selon l'une quelconque des revendications 1 à 7.

9. Procédé de formation d'une interface de phase corps solide-électrolyte sur une électrode d'une cellule électrochimique comprenant une anode, une cathode et un électrolyte, dans lequel la cellule est exploitée en utilisant un électrolyte selon l'une quelconque des revendications 1 à 7.

10. Utilisation d'un composé selon la formule générale (1) telle qu'indiquée ci-après : 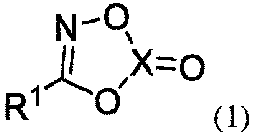 dans laquelle :
X est C ;
R¹ est choisi dans le groupe comprenant CN, alkyle en C₁-C₁₀, cycloalkyle en C₃-C₇ et/ou aryle en C₆-C₁₀, les groupes alkyle, cycloalkyle et aryle étant chacun non substitués ou substitués une fois ou plusieurs fois avec au moins un substituant choisi dans le groupe comprenant F, alkyle en C₁₋₄ et/ou CN, et
dans un accumulateur d'énergie électrochimique, notamment un supercondensateur ou un accumulateur d'énergie électrochimique à base d'ions lithium, en tant qu'additif d'électrolyte pour la formation d'une interface de phase corps solide-électrolyte.
